# EUROPÄISCHE PATENTSCHRIFT

(11) **EP 0 402 885 B1**
(45) Veröffentlichungstag und Bekanntmachung des Hinweises auf die Patenterteilung: **27.04.1994**
(21) Anmeldenummer: 90111166.6
(22) Anmeldetag: 13.06.1990
(51) Int. Cl.: A61M 25/00

(54) **Flexibler Sondenkanal**
Flexible probe duct
Canal de sonde flexible

(30) Priorität: 14.06.1989 DE 3919441
(43) Veröffentlichungstag der Anmeldung: 19.12.1990
(73) Patentinhaber: Richard Wolf GmbH, 75438 Knittlingen (DE)
(72) Erfinder: Heimberger, Rudolf, D-7519 Oberderdingen (DE)
(74) Vertreter: Wilcken, Thomas, Dipl.-Ing.

(56) Entgegenhaltungen:
- EP-A- 0 029 185
- WO-A-88/03421
- AT-B- 362 048
- DE-A- 408 449
- US-A- 1 888 349

## Beschreibung

Die Erfindung betrifft einen Sondenkanal für flexible Endoskope, aus einem flexiblen Werkstoff zum Hindurchleiten Von flexiblen Hilfsinstrumenten, beispielsweise Zangen, Elektroden oder dergleichen, sowie zum Zu- als auch zum Abführen einer Spülflüssigkeit, wobei der Sondenkanal als Schlauch mit einer glatten Innenwandfläche und mit einer äußeren Mantelfläche in Form einer Strukturierung ausgebildet ist.

Für den vorstehend angedeuteten Zweck werden üblicherweise hochflexible Kunststoffschläuche verwendet. Bei diesen Schläuchen besteht jedoch die Gefahr, daß sie beim Reinigen unter Anlegen eines Unterdruckes kollabieren oder andererseits unter Überdruck beschädigt werden können. Des weiteren besteht bei hochflexiblen Materialien die Gefahr, daß das Einführen eines scharfkantigen, geringer flexiblen Gegenstandes, wie beispielsweise einer Zange, in einen Schlauch dadurch zu dessen Beschädigung führen kann; weil solche Gegenstände nur schwer dem flexiblen Sondenkanal folgen können und diesen seitlich beanspruchen und hierbei möglicherweise sogar durchstoßen. Um diesen Nachteilen abzuhelfen, hat man diese bekannten Sondenkanäle mit zusätzlichen Metallwendeln verstärkt, was jedoch eine schwerere Auslenkbarkeit insbesondere des distalen Endes eines derartigen Sondenkanals mit sich bringt.

Durch die EP-A-0 029 185, WO-A-88/03421 und die AT-B-362 048 sind Sondenkanäle der einleitend angeführten Art bekannt, die als Katheter verwendet werden. Diese Sondenkanäle weisen jedoch aufgrund ihrer besonderen äußeren Strukturierung nur eine begrenzte Flexibilität auf. Ein weiterer Sondenkanal ist in der DE-A-408 449 beschrieben. Dieser Sondenkanal weist einen Einführungsschaft auf, der auf seiner Außenfläche mit Wellungen kugelförmiger Art versehen ist. Der Sondenkanal ist auf seiner gesamten Länge starr, wobei er ein gebogenes Vorderende aufweisen kann. In der US-A-1 888 349 ist ein Katheter beschrieben, der einen Schaft aus Weichgummi aufweist. Dieser Schaft besteht aus einem proximalen Glattschaftteil und einem distalen Ringnutschaftteil, wobei der letztere im Durchmesser größer ist. Die Nuten des Ringnutschaftteiles sind im Querschnitt eckig ausgebildet, denn sie dienen zum Aufnehmen und Festhalten eines Gleitmittels salbenförmiger Konsistenz, welches das Einführen des Katheters in eine Körperhöhle des Patienten erleichtern soll.

Die Aufgabe der Erfindung besteht in der Verbesserung eines Sondenkanals der einleitend angeführten Art für ein flexibles Endoskop, wobei der Sondenkanal einerseits eine relativ hohe Flexibilität aufweist und andererseits so stabil ist, daß Beschädigungen der genannten Art vermieden werden, der haltbar ist und leicht gereinigt werden kann sowie an wenigstens einem Ende eine erhöhte Stabilität aufweist.

Diese Aufgabe ist erfindungsgemäß durch die Merkmale des Patentanspruchs 1 gelöst.

Durch die erfindungsgemäße Ausbildung des Sondenkanals läßt sich einerseits die gewünschte hohe Flexibilität erreichen, da diese im wesentlichen von Einschnürungen, d.h. den relativ eng bei einanderliegenden Bereichen reduzierter Wandstärke bestimmt ist, andererseits aber auch die erforderliche Stabilität erzielen, die sich aus der armierenden Wirkung der jeweils benachbarten Wülste, d.h. der Bereiche größerer Wandstärke, ergibt Ferner ist eine gute Reinigbarkeit des Innenraums des Sondenkanals beim Reinigen des flexiblen Endoskopes erzielbar. Durch den ausgerundeten Nutgrund werden Kerbwirkungen vermieden, somit ist die Haltbarkeit des Sondenkanals erhöht. Schließlich ist eine Erhöhung der Stabilität des Sondenkanals an wenigstens einem seiner beiden Endbereiche erzielt, da die Tiefe der Nuten in dem Endbereich derart verringert ist, daß der betreffende Endbereich über eine bestimmte Länge eine glatte Mantelfläche mit der maximalen Wandstärke aufweist.

Eine bevorzugte Ausgestaltung des Sondenkanals besteht darin, daß die Strukturierung der äußeren Mantelfläche durch wenigstens eine wendelförmig verlaufende Nut gebildet ist.

Die Erfindung ist nachstehend anhand eines in der anliegenden Zeichnung dargestellten Ausführungsbeispieles näher erläutert. Es zeigen:
- Figur 1: das Ausführungsbeispiel des Sondenkanals in teilweise geschnittener, dessen Flexibilität aufzeigender Darstellung,
- Figur 2: ein Teilstück des Sondenkanals nach Figur 1 in teilweise geschnittener und vergrößerter Darstellung,
- Figur 3: ein Teilstück des Sondenkanals entsprechend Figur 2 in einer modifizierten Ausführungsform.

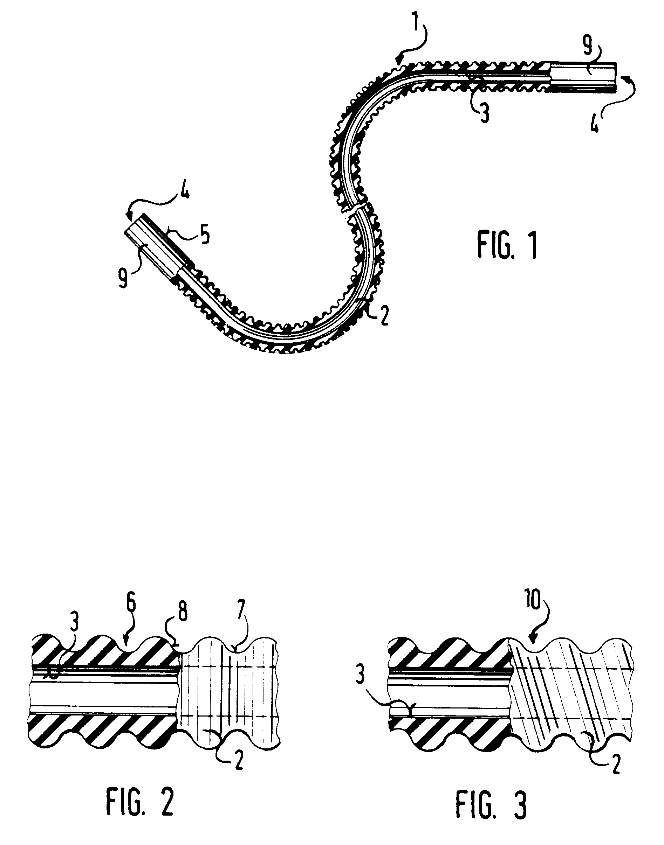

Der Sondenkanal 1 nach den Figuren 1 und 2 ist als Schlauch 2 aus einem flexiblen Kunststoff, beispielsweise Teflon, gefertigt, welcher Werkstoff aufgrund seiner antiadhäsiven Eigenschaften grundsätzlich ein leichtes Einschieben eines Hilfsinstrumentes ermöglicht sowie auch eine problemlose Reinigung gestattet. Der gezeigte Sondenkanal 1 weist eine glatte Innenwandfläche 3 auf und ist mit Ausnahme der beiden Endbereiche 4 an seiner äußeren Mantelfläche 5 strukturiert ausgeführt.

Diese Strukturierung ergibt sich, wie es am besten aus Figur 2 ersichtlich ist, aus einer Vielzahl radialer Nuten 6, in deren Bereich die Wandstärke des Schlauches 2 reduziert ist. Somit ist eine Vielzahl von zueinander beabstandeten und im wesentlichen radialen Stützwülsten ausgebildet, wobei das Verhältnis zwischen Bereichen größter und kleinster Wandstärke des Schlauches etwa 2:1 ist und die Wulstteilung etwa der doppelten größten Wandstärke entspricht.

Der Grund 7 der Nuten 6 ist ausgerundet, und die Nutflanken 8 sind an ihrem Übergang in die äußere Mantelfläche 5 gerundet ausgebildet. Zu den beiden Endbereichen 4 des Schlauches 2 hin nimmt die Tiefe der Nuten 6 beispielsweise stetig ab, so daß unstrukturierte Endstücke 9 entstehen, die eine Wandstärke aufweisen, die derjenigen im Bereich der Wülste zwischen den Nuten 6 entspricht. Mindestens ein Endbereich 9 des Sondenkanals 1 ist derart ausgebildet.

Gemäß der Ausführungsform nach Figur 3 ist die Strukturierung der Mantelfläche 5 des Sondenkanals 1 durch eine wendelförmig verlaufende Nut 10 erzeugt, die auch mehrgängig ausgeführt sein kann.

## Patentansprüche

1. Sondenkanal (1) für flexible Endoskope, aus einem flexiblen Werkstoff zum Hindurchleiten von flexiblen Hilfsinstrumenten, beispielsweise Zangen, Elektroden oder dergleichen, sowie zum Zu- als auch zum Abführen einer Spülflüssigkeit, wobei der Sondenkanal (1) als Schlauch (2) mit einer glatten Innenwandfläche (3) und mit einer äußeren Mantelfläche (5) in Form einer Strukturierung ausgebildet ist, dadurch gekennzeichnet,
daß die Strukturierung aus einer Vielzahl von zueinander beabstandeten und im wesentlichen radialen Stützwülsten besteht, derart, daß das Verhältnis zwischen Bereichen größter und kleinster Wandstärke des Schlauches (2) etwa 2:1 ist und die Wulstteilung etwa der doppelten größten Wandstärke des Schlauches entspricht,
daß der Grund (7) zwischen den Stützwülsten gerundet ausgebildet ist,
daß sich die Tiefe der zwischen den Stützwülsten befindlichen Nuten (6; 10) im Bereich mindestens eines der beiden Enden (4) des Schlauches (2) verringert und daß das betreffende Ende einen über eine bestimmte Länge unstrukturierten Endabschnitt (9) aufweist.

2. Sondenkanal nach Anspruch 1, dadurch gekennzeichnet, daß die Strukturierung der äußeren Mantelfläche (5) durch wenigstens eine wendelförmig verlaufende Nut (10) gebildet ist.

## Claims

1. A probe canal (1) for flexible endoscopes, of a flexible material for the passing through of flexible auxiliary instruments, for example forceps, electrodes or the like, and also for the supply and removal of a flushing fluid, in which the probe canal (1) is constructed as a tube (2) with a smooth inner wall surface (3) and with an outer covering surface (5) in the form of a structuring, characterised in that
the structuring consists of a plurality of substantially radial support beads, arranged at a distance from each other, such that the ratio between regions of greater and smaller wall thickness of the tube (2) is approximately 2:1 and the bead spacing corresponds approximately to twice the greatest wall thickness of the tube,
that the base (7) between the support beads is constructed so as to be rounded,
that the depth of the grooves (6; 10) situated between the support beads is reduced in the region of at least one of the two ends (4) of the tube (2) and that the respective end has an end section (9) which is unstructured over a particular length.

2. A probe canal according to Claim 1, characterised in that the structuring of the outer covering surface (5) is formed by at least one groove (10) running in a spiral shape.

## Revendications

1. Canal de sonde (1) pour un endoscope flexible, réalisé en un matériau flexible et servant au passage d'instruments auxiliaires flexibles, par exemple des pinces, des électrodes ou analogues, ainsi que pour l'amenée et l'évacuation d'un liquide de lavage, le canal de sonde (1) étant réalisé sous la forme d'un tuyau (2) possédant une surface de paroi inférieure (3) lisse, et une surface enveloppe extérieure (5) sous la forme d'une configuration structurée, caractérisé par le fait
que la configuration structurée est constituée par une multiplicité de bourrelets de support distants les uns des autres et sensiblement radiaux, de sorte que le rapport entre les zones possédant l'épaisseur de paroi maximale et les zones possédant l'épaisseur de paroi minimale du tuyau (2) est égale à environ 2:1 et que le pas de répartition des bourrelets correspond approximativement au double de l'épaisseur de paroi maximale du tuyau,
que le fond (7) présent entre deux bourrelets de support est arrondi,
que la profondeur des gorges (6,10) situées entre les bourrelets de support est réduite au niveau d'au moins l'une des deux extrémités (4) du tuyau (2) et que l'extrémité considérée possède une section d'extrémité (9) non structurée sur une longueur déterminée.

2. Canal de sonde selon la revendication 1, caractérisé en ce que la configuration structurée de la surface enveloppe extérieure (5) est formée par au moins une gorge de forme hélicoïdale (10).
